# EUROPÄISCHE PATENTANMELDUNG

(11) **EP 0 714 637 A2**
(43) Veröffentlichungstag der Anmeldung: **05.06.1996**
(21) Anmeldenummer: 95117295.6
(22) Anmeldetag: 03.11.1995
(51) Int. Cl.: A61B 19/08

(54) **Operationsabdecktuch**

(30) Priorität: 07.11.1994 DE 9417818 U; 31.12.1994 DE 9420953 U
(71) Anmelder: MEWA Textil-Service AG, D-65189 Wiesbaden (DE)
(72) Erfinder:
(74) Vertreter: Tergau, Enno, Dipl.-Ing.

(57) **Zusammenfassung**

Bei einem Abdecktuch für medizinische Operationen an Extremitäten mit einer Durchstecköffnung (6) für die Extremität ist die Durchstecköffnung (6) Bestandteil einer aus einer gummiartigen Kunststoffolie bestehenden Nahfeldabdeckung (1). Die Nahfeldabdeckung (1) überdeckt mit ihrem Umfangsrand (7) ein einen größeren Wirkquerschnitt als die Durchstecköffnung (6) aufweisendes Durchgangsloch (8) einer Umfeldabdeckung (2) von wesentlich größerem Format als die Nahfeldabdeckung (1). Die Nahfeldabdeckung (1) ist im Auflagebereich ihres Umfangsrandes (7) durch eine lösbare Haftschlußverbindung mit der Umfeldabdeckung (2) keimdicht und lösbar verbunden. Die Nahfeldabdeckung (1) ist ein Einwegartikel, während die Umfeldabdeckung durch Waschen, Sterilisieren und durch Aufkleben einer neuen Nahfeldabdeckung (1) wiederverwendbar ist.

## Beschreibung

Die Erfindung betrifft eine Abdeckung mit den im Oberbegriff des Anspruches 1 aufgeführten Merkmalen.

Derartige Operationsabdeckungen - nachstehend auch kurz "OP-Abdeckung" genannt - sind herkömmlich Einwegartikel. Sie bestehen aus einem keim- und flüssigkeitsdichten Werkstoff und sind im wesentlichen einstückig ausgebildet. Der Werkstoff kann auch ein Laminat, z.B. beschichtets Papier, sein.

Der Erfindung liegt die Aufgabe zugrunde, eine speziell für medizinische Operationen an Extremitäten wie insbesondere Armen und Beinen geeignete OP-Abdeckung zu schaffen, die den Operationsgegenstand` nämlich die durch die Durchstecköffnung hindurch auf die Oberseite der Abdeckung geführte Extremität, keimdicht gegenüber dem Patienten, seiner Bekleidung und/oder einer anderweitigen Abdeckung abschirmt, einfach bedienbar ist und umweltschonend entsorgt werden kann. Diese Aufgabe wird durch Anspruch 1 gelöst. Im Kern beinhaltet die Erfindung den Gedanken, die OP-Abdeckung in eine Nahfeldabdeckung und eine Umfeldabdeckung aufzuteilen, die während der bestimmungsgemäßen Benutzung der OP-Abdeckung ausreichend fest und keimdicht miteinander verbunden sind, jedoch nach der bestimmungsgemäßen Benutzung einfach voneinander gelöst werden können. Die in ihren Abmessungen auf das Notwendige, nämlich auf den primär abzuschirmenden operationsnahen Bereich, begrenzte Nahfeldabdeckung ist dabei besonders bedienungs- und benutzungsfreundlich ausgebildet, indem sie aufgrund der Dimensionierung ihrer Durchstecköffnung und ihres Werkstoffes selbsttätig eine enge, manschettenartige und damit flüssigkeitsdichte Anlage an der durch sie hindurchreichenden Extremität gewährleistet, nach dem Gebrauch jedoch als Einwegartikel umweltschonend entsorgt wird, während die wesentlich voluminösere Umfeldabdeckung durch einen Waschvorgang regenerierbar und durch haftschlüssiges Aufsetzen einer neuen Nahfeldabdeckung einem erneuten Gebrauch zuführbar ist.

Die nach einem bestimmungsgemäßen Gebrauch zu erfolgende Lösung der Nahfeldabdeckung von der Umfeldabdeckung läßt sich durch Verwendung eines mittels Wärme lösbaren bzw. inaktivierbaren Klebers besonders umweltfreundlich und zerstörungsfrei bewerkstelligen.

Durch die Verwendung einer elastisch aufweitbaren gummiartigen Kunststoffolie für die Nahfeldabdeckung ist bei entsprechender Dimensionierung der Durchstecköffnung gewährleistet, daß sich die Nahfeldabdeckung eng an Extremitäten unterschiedlicher Durchmesser anlegt, wobei wegen ihres Einwegcharakters auch eine Überdehnung ihrer Elastizität in Kauf genommen werden kann.

Die Mehreck- und insbesondere die quadratische Umrißform der Nahfeldabdeckung nach Anspruch 8 oder 9 erleichtert ihr beschädigungsfreies Lösen nach einem bestimmungsgemäßen Gebrauch, weil sie eine Griffecke zur Verfügung stellt.

Während die Nahfeldabdeckung aus einem einheitlichen Werkstoff, insbesondere einer Kunststoffolie, besteht, ist die Umfeldabdeckung zweckmäßig durch einen Schichtstoff gebildet, der nach den Ansprüchen 10 bis 13 oberseitig ein saugfähiges Polyestergewirke und darunter eine mikroporöse Membrane z.B. aus flüssigkeitsundurchlässigem PTFE aufweist, die jedoch die Körperfeuchtigkeit in Form von Wasserdampf entweichen läßt. Letztere erhöht den Klimakomfort des Patienten. Weiterhin enthält die Umfeldabdeckung zweckmäßig auf ihrer dem Patienten zugewandten Unterseite ebenfalls eine Schicht aus jedoch schwächer flüssigkeitsabsorbierendem Polyestergewirke.

Die Nahfeldabdeckung liegt auf der Oberseite der Umfeldabdeckung auf. Um trotz der Saugfähigkeit des die Oberseite bildenden Polyestergewirkes eine Keimdichtigkeit der Haftschluß-, insbesondere der Klebeverbindung, mit der Nahfeldabdeckung zu gewährleisten, ist die Ausgestaltung nach Anspruch 14 vorgesehen.

Die Umfeldabdeckung ist schwer entflammbar und mit über 120° sterilisierbar, so daß eine vollwertige Regenerierung durch Waschen und anschließendes Sterilisieren gewährleistet ist.

Die Umfeldabdeckung ist hinsichtlich ihrer Umrißform so formatiert, daß eine vollständige Patientenabdeckung möglich ist (Ansprüche 17 und 18). Zur besseren Handhabung dient die weitere Ausgestaltung der Umfeldabdeckung nach Anspruch 19 ff. Die durch das Barriergewebe gebildeten Randstreifen wirken gegenüber dem Zentralteil der Umfeldabdeckung als Sterilfeld und Trockenkeimsperre. Die Randstreifen enthalten wenigstens auf einer Seite Fixationsösen z.B. zum Einhängen an einem für das Aufhängen von Fusionsflaschen o.dgl. bestimmten Stativ.

Gemäß besonderer Ausführungsform sind allseitig Randstreifen vorgesehen. Zweckmäßig sind die Randstreifen von gleicher Breite. Das Randstreifengewebe ist ein Mikrofilamentgarn-Gewebe, welches nachträglich mit einem texturierten Griff, z.B. durch Schmirgeln versehen ist. Die dadurch erzielte Verbesserung der Griffigkeit erleichtert die Handhabbarkeit der Abdeckung sowohl bei der Operation als auch beim Zusammenlegen nach dem Waschen bzw. Reinigen und der Sterilisation, d.h. am Ende des Recycling-Vorganges. Durch den allseits angebrachten Randstreifen ist der Zentralbereich von einem Sterilfeld umgeben, welches aus insbesondere nichtsaugendem Gewebe besteht. Die Vorteilhaftigkeit der nichtsaugenden Eigenschaft des Gewebes ergibt sich aus der Überlegung, daß es die Randkanten sind, die bei einer Operation den dem Fußboden des Operationsraumes nächsten Bereich der Abdeckung bilden. Der Fußboden ist häufig von Flüssigkeit bedeckt, die vom zentralen Operationsbereich, hier also von der Nahfeldabdeckung, möglichst ferngehalten werden muß.

Schließlich betrifft die Erfindung die sogenannte Faltlogistik der Abdeckung. Hier geht es darum, mit möglichst wenig und gezielten Griffen ein Zusammenlegen der in einen neu gebrauchsfähigen Zustand versetzten Abdeckung so zu ermöglichen, daß die der Operationsstelle zugewandte, also dem Körper des Patienten abgewandte Oberfläche der Abdeckung um so besser gegen die Einwirkung äußerer Verunreinigungen geschützt ist, je näher der Oberflächenbereich dem Durchgangsloch 8 der Nahfeldabdeckung 1 steht. Die Faltlogistik ist also nach dem Gesichtspunkt orientiert, daß die dem Körper des Patienten abgewandte bzw. der Operationsstelle an der Extremität zugewandte Oberfläche insbesondere der Nahfeldabdeckung in paketiertem Zusammenleg-Zustand durch möglichst viele Faltlagen nach außen abzudecken bzw. abzuschirmen. Die durch Striche oder Pfeile markierten Randbereiche halten einen maximalen Abstand zur Durchstecköffnung 6, d.h. zu dem unbedingt sicher steril zu haltenden Bereich der Abdeckung 1. Das Zusammenlegen erfolgt in aller Regel durch zwei Personen, die jeweils an einander gegenüberliegenden Randkanten im Bereich der Strich- oder Pfeilmarkierung angreifen und das Zusammenlegen zunächst in der einen und dann in der anderen Richtung der Randkanten bewerkstelligen.

Ein weiteres Erfindungsmerkmal besteht in der Aufbringung eines _{"}Useometers" auf der in paketierter Zusammenlegstellung außenliegenden Paketoberfläche. Auf dem als Rasterfeld ausgebildeten Useometer wird jeweils der Vollzug eines Recycling-Zyklus nach dem Zusammenlegen der Abdeckung durch eine Markierung eines Karos, z.B. durch dessen Auskreuzen gekennzeichnet. Dadurch ist in besonders signifikanter Weise die Anzahl der Recycling-Vorgänge erkennbar, denen die Abdeckung bereits ausgesetzt gewesen ist. Das erleichtert eine Kontrolle des Gebrauchs- bzw. Nutzungswertes der Abdeckung. Schließlich ist auf der Oberfläche des Abdeckungspaketes ein Richtungspfeil zur zielsicheren Ausrichtung des Abdeckungspaketes auf dem Körper des Patienten vor dem Auseinanderfalten der Abdeckung aufgebracht. Damit ist sichergestellt, daß das Ausbreiten der Abdeckung mit möglichst wenig Griffen auf dem Körper des Patienten ohne nachträgliches Ausrichten vorgenommen werden kann, welches die Gefahr einer Verunreinigung oder Kontaminierung mit Keimen minimiert. Diese Faltlogistik stellt auch sicher, daß das Operationspersonal die Abdeckung bei ihrem Ausbreiten nur an den markierten, vom Operationsnahfeld entfernten Stellen ergreift und somit für eine höchstmögliche Keimfreiheit im Operationsnahfeld sorgt.

Der Gegenstand der Erfindung wird anhand eines in den Figuren dargestellten Ausführungsbeispiels erläutert. Dabei zeigen
- Fig. 1: eine Draufsicht auf die Oberseite der OP-Abdeckung,

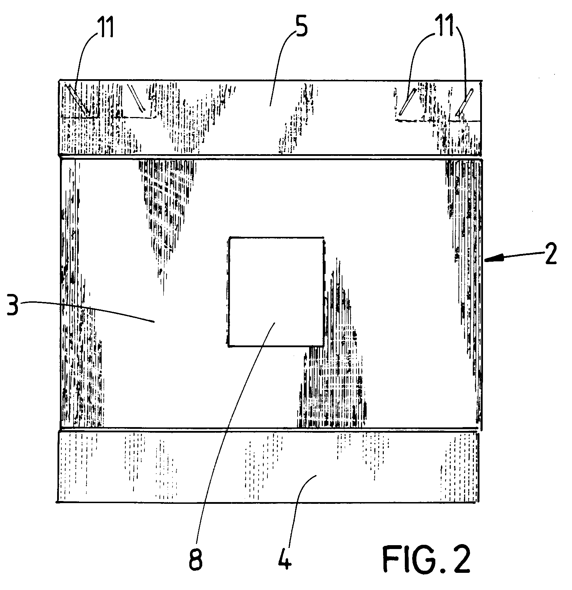

- Fig. 2: eine Draufsicht auf die Unterseite der OP-Abdeckung gem. Fig. 1 und
- Fig. 3: eine schematische Darstellung der OP-Abdeckung im Applikationszustand, nämlich bei durch die Durchstecköffnung der Nahfeldabdeckung auf die Oberseite der OP-Abdeckung durchreichender Extremität, hier z.B. eine Patientenhand.
- Fig. 4: eine Draufsicht auf eine modifizierte Ausführungsform der OP-Abdeckung mit lediglich schematisiert dargestellter Nahfeldabdeckung, indessen einem umlaufenden Randstreifenbereich und mit Markierungen für die Faltlogistik.
- Fig. 5: eine perspektivische Ansicht der Abdeckung in aus einer paketierten Zusammenlegstellung teilweise auseinandergelegter Stellung.
- Fig. 6: eine perspektivische Darstellung der in einer Richtung vollständig aus einer paketierten Zusammenlegstellung auseinandergelegten Abdeckung mit in die andere, rechtwinklig dazu verlaufende Auseinanderlegrichtung teilweise ausgebreiteter Position.
- Fig. 7: eine Draufsicht auf einen Ausschnitt der Oberfläche des Abdeckungspaketes mit einem _{"}Useometer" und einem Richtungspfeil zur Erleichterung der Ausrichtung des Abdeckungspaketes am Patienten vor dem Auseinanderfalten.

Die tuchartige OP-Abdeckung besteht im wesentlichen aus der zentral positionierten Nahfeldabdeckung 1 und der diese tragenden Umfeldabdeckung 2. Die Umfeldabdeckung 2 wiederum enthält einen Zentralbereich 3 mit zwei einander gegenüberliegenden Randstreifen 4,5.

Die Nahfeldabdeckung 1 besteht aus einem flüssigkeits- und feuchtigkeitsundurchlässigen Werkstoff, insbesondere aus einer gummiartigen Kunststoffolie. In ihrem Zentralbereich enthält sie eine Durchstecköffnung 6 vorzugsweise in Form etwa einer Manschette. Die Nahfeldabdeckung 1 überdeckt mit ihrem Umfangsrand 7 das Durchgangsloch 8 der Umfeldabdeckung 2, die sich im wesentlichen über den peripheren Bereich der Nahfeldabdeckung 1 hinaus nach außen erstreckt. Der Zentralbereich 3 der Umfeldabdeckung 2 ist mit einer saugfähigen Oberfläche ausgestattet. Die Umfeldabdeckung 2 ist waschbar und von wesentlich größerem, in der Darstellungsebene der Figuren wirksamem Format als die Nahfeldabdeckung 1.

Das vorzugsweise quadratische Durchgangsloch 8 in der Umfeldabdeckung 2 weist einen wesentlich größeren Wirkquerschnitt auf als die Durchstecköffnung 6 der Nahfeldabdeckung 1. Dadurch besteht ein ausreichend großer Abstand zwischen der Durchstecköffnung 6 und dem Umfangsrand 7 als Träger der Haftschlußverbindung zwischen Nahfeldabdeckung 1 und Umfeldabdeckung 2. In diesem Abstandsbereich kann sich die Eigenelastizität des gummiartigen Kunststoffwerkstoffes der Nahfeldabdeckung 1 voll auswirken, um eine dichte, manschettenartige Umfassung der durch die Durchstecköffnung 6 hindurchreichenden Extremität zu gewährleisten. Die Haftschlußverbindung im Bereich des Umfangsrandes 7 zwischen Nahfeldabdeckung 1 und Umfeldabdeckung 2 ist eine keimdichte Klebeverbindung. Der Kleber ist zweckmäßig durch Wärme inaktivierbar, so daß es für die nachträgliche Lösung der Nahfeldabdeckung von der Umfeldabdeckung nicht des Einsatzes eines speziellen Lösungsmittels bedarf. Es braucht dann nur Wärme appliziert zu werden, wie dies z.B. auch durch einen Vorwaschvorgang geschehen kann. Die gummiartige Nahfeldabdeckung ist aus einem solchen Werkstoff gefertigt, der eine ausreichende elastische oder sogar plastische Aufweitbarkeit der Durchstecköffnung 6 ermöglicht. Die Durchstecköffnung 6 ist zweckmäßig kreisförmig mit einem Kreisdurchmesser zwischen ca. 4 und 7 cm. Zweckmäßig werden Nahfeldabdeckungen mit abgestuften Öffnungsquerschnitten zur Verfügung gestellt, je nachdem, ob die zu operierende Extremität ein Arm oder ein Bein ist. Hier haben sich Abmessungen von 4,2 und 6,5 cm als vorteilhaft erwiesen.

Die Nahfeldabdeckung 1 weist eine quadratische Umrißform auf. Sie bietet dadurch ausgeprägte Ecken 9 zum einfacheren Ergreifen von Hand, wenn nach Gebrauch die Lösung der Nahfeldabdeckung von der Umfeldabdeckung 2 erwünscht ist. Die Quadratform hat zweckmäßig eine Kantenlänge von etwa 35 cm.

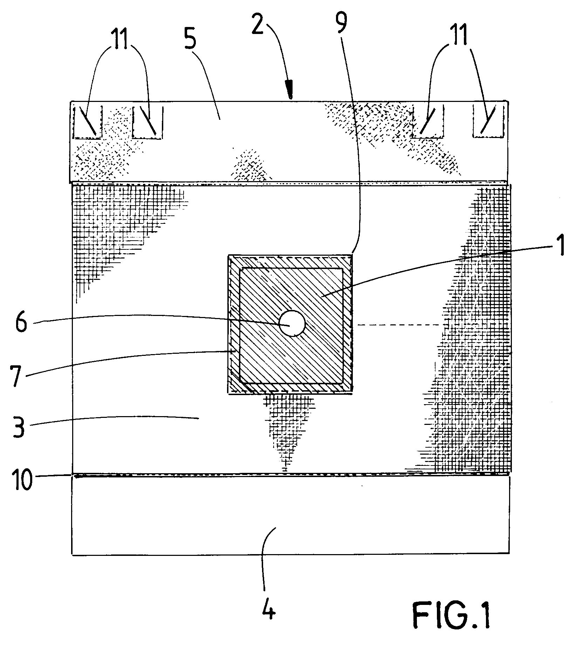

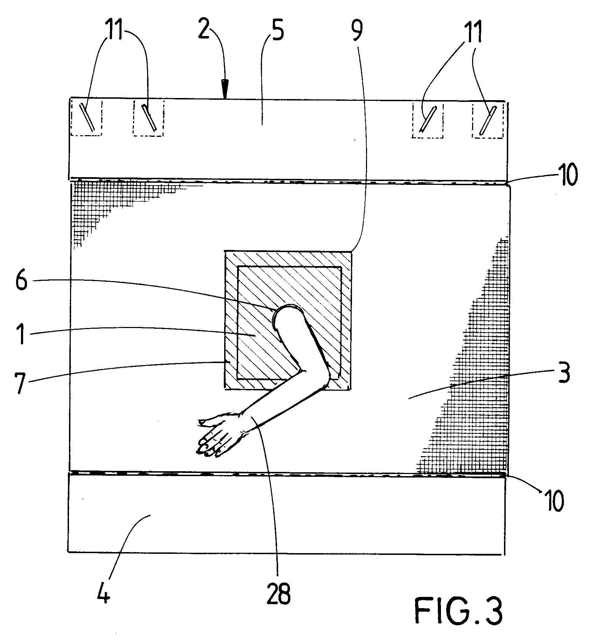

Die Umfeldabdeckung ist durch einen dreischichtigen Schichtstoff gebildet. Die Oberschicht (sichtbar in den Fig. 1 und 3) ist durch ein saugfähiges Polyestergewirke gebildet. Die Mittelschicht ist eine mikroporöse Membrane aus flüssigkeitsundurchlässigem PTFE oder aus einem wirkungsgleichen Werkstoff, die aber die Körperfeuchtigkeit in Form von Wasserdampf von der Unterseite her entweichen läßt, was dem Klimakomfort des Patienten dienlich ist. Die Unterseite besteht wiederum aus einem saugfähigen Polyestergewirke. Dieses ist aber zweckmäßig so eingestellt, daß seine Saugfähigkeit bzw. Absorptionsfähigkeit schwächer bzw. geringer ist als die des die Oberseite bildenden Polyestergewirkes. Jedenfalls ist der den Zentralbereich 3 bildende Schichtwerkstoff flüssigkeitskeimdicht.

Die oberseitige Textilschicht im Überdeckungsbereich des Umfangsrandes 7 der Nahfeldabdeckung 1 ist durch eine Imprägnierung hydrophob ausgebildet, so daß einerseits ein sicherer Haftgrund für den Kleber vorliegt, andererseits aber auch eine Keimdichtigkeit der Klebeverbindung gewährleistet ist. Diese Dichtheit ist auch gegenüber einer möglichen Keimübertragung mit Flüssigkeiten wirksam.

Die Umfeldabdeckung 2 ist schwer entflammbar und mit über 120°C sterilisierbar.

Die Umfeldabdeckung 2 weist eine rechteckige, insbesondere eine quadratische Umrißform auf mit vorzugsweise einer Außenabmessung von ca. 250 x 250 cm, insbesondere von 240 x 250 cm. Die Umfeldabdeckung 2 enthält an ihrem oberen und unteren Rand zwei einander gegenüberliegende Randstreifen 4,5 aus einem Barriergewebe aus Mikrofasern. Es handelt sich um ein Polyestergewebe, dem etwa 1% Karbon beigegeben sein kann. Dieses Gewebe ist antistatisch und partikelfrei. Die Randstreifen 4,5 dienen als Sterilfeld und Trockenkeimsperre. Sie sind durch Nahtverbindungen 10 an den Zentralbereich 3 angenäht. Mindestens ein Randstreifen 5 kann mit Fixationsösen 11 versehen sein.

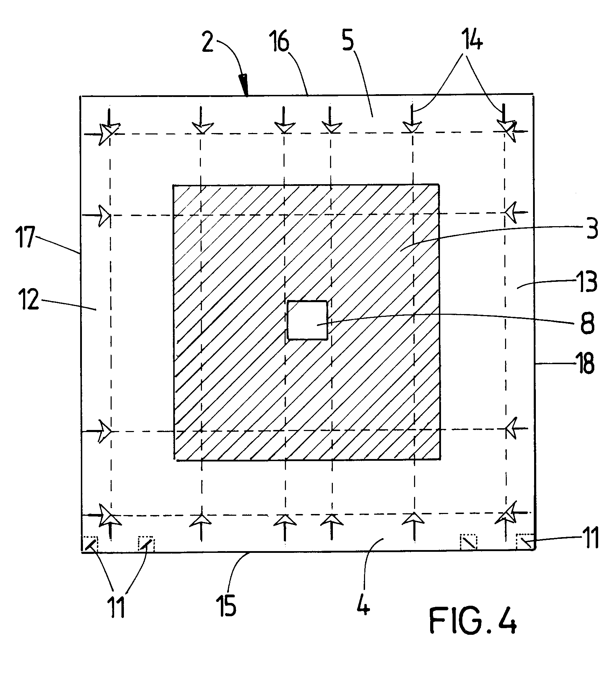

Beim Ausführungsbeispiel gemäß Fig. 4 ist der Zentralbereich 3 allseitig von einem Randstreifen 4,5 bzw. 12,13 umgeben. Dabei verlaufen die Randstreifen 4,5 und 12,13 zueinander parallel. Die Randstreifenpaare 4,5 und 12,13 sind - bezogen auf ihre Längserstreckung - rechtwinklig zueinander ausgerichtet. Das Randstreifengewebe ist ein Mikrofilamentgarn-Gewebe, welches durch nachträgliches Schmirgeln zur Verbesserung der Handhabbarkeit mit einem texturierten Griff versehen ist. Das Einarbeiten von Carbonfäden vermittelt dem den Zentralbereich umgebenden Sterilfeldbereich ein antistatisches Verhalten.

Die Abdeckung ist in ihren Randbereichen allseitig mit Faltmarkierungen 14 zur Erleichterung der Positionierung der Griffalten zum Zwecke der Paketierung der Abdeckung versehen. Diese Faltmarkierungen 14 sind durch eine Gerade (Strich) oder einen Pfeil gebildet, die den Sollverlauf einer Griffalte markieren und damit die Fingergriffrichtung zur Faltenbildung erleichtern. Einander gegenüberliegenden Randstreifen 4,5 bzw. 12,13 zugeordnete Faltmarkierungen 14 liegen einander paarweise gegenüber. Sie kennzeichnen die beiden Enden jeweils einer Griffalte und sind dementsprechend dem Bereich der Randkanten 15-18 zugeordnet.

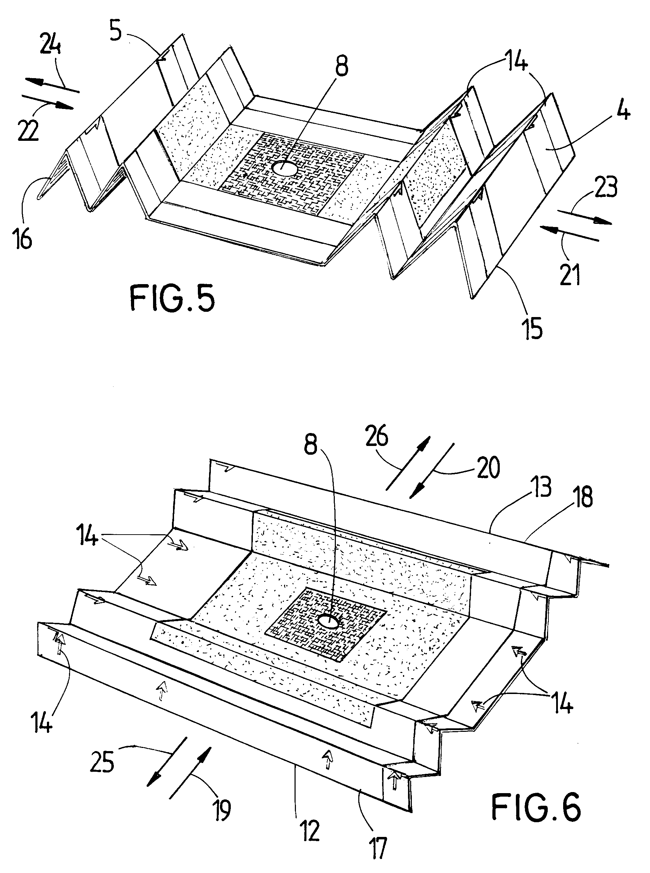

Die Faltmarkierungen 14 sind auf der dem Patientenkörper abgewandten bzw. der dem Operationsbereich 28 zugewandten Oberfläche der Abdeckung angeordnet. Das Zusammenlegen der Abdeckung zunächst in der einen Richtung 19,20 (Fig. 6) und dann in den anderen Richtungen 21,22 (Fig. 5) erfolgt nach Art eines Leporello und zwar zunächst in den Zusammenlegrichtungen 19,20 und dann in den dazu rechtwinkligen Zusammenlegrichtungen 21,22 beginnend von den jeweils außenliegenden Randkanten 15-18 zum Durchgangsloch 8 der Nahfeldabdeckung 1 gerichtet. Beim Zusammenlegen wird die Abdeckung nur im Bereich der Randkanten 15-18 der Randstreifen 4,5 bzw. 12,13 ergriffen, wobei der Verlauf der Längsrichtung der Falten durch die Richtungslinie bzw. den Pfeilschaft der Faltmarkierung 14 markiert ist. Das Zusammenlegen erfolgt durch zwei Personen, die die besagten Randstreifen 4,5 bzw. 12,13 in deren Randkantenbereich ergreifen, dort unter Vorgabe der Richtung des Faltenverlaufes mit den Fingerspitzen erfassen und zur Bildung nach Art einer Bügelfalte falzen. Auf diese Weise ist die Abdeckung zu Zwecken der Lager- und Transportlogistik zu einem Paket (nicht dargestellt) zusammenlegbar, bei welchem die dem Körper des Patienten abgewandte Außenseite, insbesondere in ihrem der Nahfeldabdeckung 1 nahen Bereich mittels mehrerer Faltlagen abgedeckt und somit gegen Verunreinigung optimal abgeschirmt ist.

Das Auseinanderlegen des Abdeckungspaketes erfolgt auf dem Körper des Patienten wiederum zunächst in den Pfeilrichtungen 23,24 und nach der vollständigen Ausbreitung in diesen Richtungen dann in dazu rechtwinklig verlaufender Auseinanderlegrichtung 25,26 entgegen den Zusammenlegrichtungen 19,20.

Um das Abdeckungspaket vor dem geschilderten Auseinanderlegen auf dem Körper des Patienten noch vor dem Auseinanderfalten bestimmungsgemäß auszurichten, trägt das Abdeckungspaket auf seiner Oberseite einen auf den Patientenkopf weisenden Richtungspfeil 27.

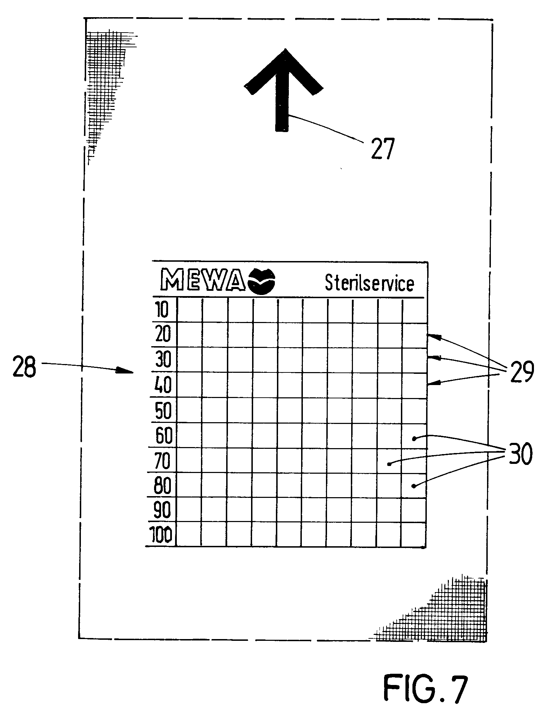

Des weiteren ist auf der Paketoberfläche (Fig. 7) ein _{"}Useometer" in Form eines Rasterfeldes 28 aufgebracht. Dieses Rasterfeld 28 dient zur Markierung von Gebrauchszyklen. Es besteht aus mehreren, insbesondere aus zehn übereinander angeordneten Feldstreifen 29 mit nebeneinander angeordnet jeweils zehn Karos 30. Jedem Feldstreifen 29 ist eine Dekadenziffer vorangestellt, in Fig. 7 untereinander die Zahlen 10,20,30 ff.

Nach einem bestimmungsgemäßen Gebrauch der OP-Abdeckung wird die Nahfeldabdeckung 1 zerstörungsfrei von der Umfeldabdeckung 2 abgezogen oder durch den Waschprozeß entfernt. Dies kann auch ohne Wärmeeinfluß möglich sein, wenn es sich um einen entsprechend eingestellten Leichtkleber handelt. Die Nahfeldabdeckung 1 wird dann als Einwegartikel entsorgt, während die Umfeldabdeckung 2 durch Waschen und Sterilisieren regenerierbar und durch anschließende Applikation einer neuen Nahfeldabdeckung 1 eine neue OP-Abdeckung bildet.

### Bezugszeichenliste

- 1: Nahfeldabdeckung
- 2: Umfeldabdeckung
- 3: Zentralbereich
- 4: Randstreifen
- 5: Randstreifen
- 6: Durchstecköffnung
- 7: Umfangsrand
- 8: Durchgangsloch
- 9: Ecke
- 10: Nahtverbindung
- 11: Fixationsöse
- 12: Randstreifen
- 13: Randstreifen
- 14: Faltmarkierung
- 15: Randkante
- 16: Randkante
- 17: Randkante
- 18: Randkante
- 19-22: Pfeilrichtung
- 23-26: Pfeilrichtung
- 27: Richtungspfeil
- 28: Operationsbereich (Fig. 3)

## Patentansprüche

1. Tuchartige Abdeckung für medizinische Operationen an Extremitäten wie Arme, Beine o.dgl. mit einer Durchstecköffnung (6) für die Extremität,
gekennzeichnet durch die Merkmale:
a) eine
- aus einem flüssigkeits- und feuchtigkeitsundurchlässigen Werkstoff, insbesondere aus einer gummiartigen Kunststoffolie bestehende,
- in ihrem Zentralbereich die Durchstecköffnung (6) in Form etwa einer Manschette enthaltende
Nahfeldabdeckung (1) überdeckt mit ihrem Umfangsrand (7) das Durchgangsloch (8) einer
- sich im wesentlichen über den peripheren Bereich der Nahfeldabdeckung (1) hinaus erstreckenden,
- mit saugfähiger Oberfläche ausgestatteten und
- waschbaren
Umfeldabdeckung (2) von größerem Format als die Nahfeldabdeckung (1),
b) die Nahfeldabdeckung (1) ist im Auflagebereich ihres Umfangsrandes (7) durch eine lösbare Haftschlußverbindung mit der Umfeldabdeckung (2) keimdicht verbunden.

2. Abdeckung nach Anspruch 1
dadurch gekennzeichnet,
daß das Durchgangsloch (8) in der Umfeldabdeckung (2) einen größeren Wirkquerschnitt aufweist als die Durchstecköffnung (6) der Nahfeldabdeckung (1),

3. Abdeckung nach Anspruch 1 oder 2,
gekennzeichnet durch
eine Klebeverbindung zwischen Nahfeld- (1) und Umfeldabdeckung (2).

4. Abdeckung nach Anspruch 3,
gekennzeichnet durch
einen mittels Wärme lösbaren Kleber.

5. Abdeckung nach einem der Ansprüche 1 bis 4,
gekennzeichnet durch
eine elastische Aufweitbarkeit der Durchstecköffnung (6) der Nahfeldabdeckung (1).

6. Abdeckung nach Anspruch 1 oder 5,
gekennzeichnet durch
eine Kreisform der Durchstecköffnung (6).

7. Abdeckung nach Anspruch 6,
dadurch gekennzeichnet,
daß der Kreisdurchmesser etwa 4-7 cm beträgt.

8. Abdeckung nach einem oder mehreren der vorhergehenden Ansprüche,
gekennzeichnet durch
eine mehreckige, vorzugsweise rechteckige und insbesondere quadratische Umrißform der Nahfeldabdeckung (1).

9. Abdeckung nach Anspruch 8,
gekennzeichnet durch
eine Kantenlänge von etwa 35 cm der Quadratform.

10. Abdeckung nach einem oder mehreren der vorhergehenden Ansprüche,
dadurch gekennzeichnet,
daß die Umfeldabdeckung (2) einen Schichtstoff enthält, insbesondere aus einem Schichtstoff besteht.

11. Abdeckung nach Anspruch 10,
gekennzeichnet durch
einen zweischichtigen Aufbau aus oberseitig einem Textilgewirke und darunter (patientenseitig) einer mikroporösen und daher wasserdampfdurchlässigen Membrane vorzugsweise aus flüssigkeitsundurchlässigem PTFE.

12. Abdeckung nach Anspruch 11,
gekennzeichnet durch
einen dreischichtigen Aufbau mit oberseitig und unterseitig einem saugfähigen Textilstoff aus Polyester und dazwischen einer mikroporösen Membrane.

13. Abdeckung nach Anspruch 12,
dadurch gekennzeichnet,
daß die Absorptionsfähigkeit der unterseitigen Textilschicht, insbesondere eines Gewirkes, geringer ist als die der oberseitigen Textilschicht.

14. Abdeckung nach einem oder mehreren der vorhergehenden Ansprüche,
dadurch gekennzeichnet,
daß die oberseitige Textilschicht im Überdeckungsbereich des Umfangsrandes (7) der Nahfeldabdeckung (1) durch eine Imprägnierung hydrophob ausgebildet ist.

15. Abdeckung nach einem der Ansprüche 10 bis 14,
dadurch gekennzeichnet,
daß der Schichtstoff schwer entflammbar und über 120°C sterilisierbar ist.

16. Abdeckung nach einem der Ansprüche 10 bis 15,
gekennzeichnet durch
eine Dichtheit des Schichtstoffes auch gegen Keime im flüssigen Medium.

17. Abdeckung nach einem oder mehreren der vorhergehenden Ansprüche,
gekennzeichnet durch
eine rechteckige, insbesondere eine quadratische Umrißform der Umfeldabdeckung (2).

18. Abdeckung nach Anspruch 17,
gekennzeichnet durch
eine Außenabmessung von ca. 250 x 250 cm, insbesondere von 240 x 250 cm.

19. Abdeckung nach einem oder mehreren der vorhergehenden Ansprüche,
dadurch gekennzeichnet,
daß die Umfeldabdeckung (2) an zwei einander gegenüberliegenden Randbereichen Randstreifen (4,5) aus einem Barriergewebe aus Mikrofasern enthält.

20. Abdeckung nach Anspruch 19,
gekennzeichnet durch
eine Streifenbreite von jeweils ca. 50 cm.

21. Abdeckung nach Anspruch 19 oder 20,
gekennzeichnet durch
allseitige Randstreifen (4,5) bzw. (12,13) aus insbesondere nichtsaugendem Gewebe

22. Abdeckung nach einem der Ansprüche 19 bis 21,
dadurch gekennzeichnet,
daß das Randstreifengewebe im wesentlichen aus Polyester besteht.

23. Abdeckung nach Anspruch 22,
gekennzeichnet durch
eine Beigabe von ca. 1% Carbonfäden.

24. Abdeckung nach einem der Ansprüche 19 bis 23,
dadurch gekennzeichnet,
daß das Randstreifengewebe ein Mikrophilamentgarn-Gewebe ist.

25. Abdeckung nach Anspruch 24,
gekennzeichnet durch
einen texturierten Griff des Mikrophilamentgarn-Gewebes.

26. Abdeckung nach einem der Ansprüche 19 bis 25,
gekennzeichnet durch
eine partikelfreie und/oder antistatische Konsistenz des Randstreifengewebes.

27. Abdeckung nach einem der Ansprüche 19 bis 26,
gekennzeichnet durch
eine Nahtverbindung zwischen den Randstreifen und dem Zentralbereich (3) der Umfeldabdeckung.

28. Abdeckung nach einem der Ansprüche 19 bis 27,
gekennzeichnet durch
Fixationsösen (11) im Bereich mindestens eines Randstreifens (4,5).

29. Abdeckung nach einem der vorhergehenden Ansprüche,
dadurch gekennzeichnet,
daß in den Randbereichen der Umfeldabdeckung allseitig Faltmarkierungen (14) für die Positionierung der Griffalten zum Zusammenlegen der Abdeckung zu einem Paket angeordnet sind.

30. Abdeckung nach Anspruch 29,
dadurch gekennzeichnet,
daß die Positionsmarkierung eine Strich- oder Pfeilmarkierung zur Kennzeichnung der Ausrichtung der Griffalten sind.

31. Abdeckung nach Anspruch 30,
dadurch gekennzeichnet,
daß gegenüberliegenden Randkanten (15-18) zugeordnete Faltmarkierungen (14) einander paarweise gegenüberliegen und die Enden jeweils einer Griffalte für das Zusammenlegen zu einem Abdeckungspaket markieren.

32. Abdeckung nach einem der Ansprüche 29 bis 31,
dadurch gekennzeichnet,
daß die Faltmarkierungen (14) auf ihrer dem Patientenkörper abgewandten Oberseite angeordnet sind.

33. Abdeckung insbesondere nach einem oder mehreren der vorhergehenden Ansprüche,
gekennzeichnet durch
ein im Paketzustand auf der Paketoberseite aufgebrachtes Rasterfeld (28) zur Markierung von Gebrauchszyklen.

34. Abdeckung nach Anspruch 33,
dadurch gekennzeichnet,
daß das Rasterfeld (28) aus mehreren, insbesondere aus zehn übereinander angeordneten Feldstreifen (29) besteht, welche nebeneinander angeordnet jeweils zehn Karos (30) enthalten.

35. Abdeckung nach Anspruch 34,
dadurch gekennzeichnet,
daß jedem Feldstreifen (29) eine Dekadenzahl vorangestellt ist.

36. Abdeckung insbesondere nach einem oder mehreren der vorhergehenden Ansprüche,
gekennzeichnet durch
einen vor dem Auseinanderfalten der Abdeckung auf den Patientenkopf ausrichtbaren Richtungspfeil (27) auf der Oberfläche des zusammengelegten Abdeckungspaketes.
